(19) Europäisches Patentamt / European Patent Office / Office européen des brevets

(11) **EP 3 108 016 B1**

(12) **FASCICULE DE BREVET EUROPEEN**

(45) Date de publication et mention
de la délivrance du brevet:
**24.10.2018 Bulletin 2018/43**

(21) Numéro de dépôt: **15709252.9**

(22) Date de dépôt: **16.02.2015**

(51) Int Cl.:
*C12R 1/865* (2006.01)   *C12N 15/04* (2006.01)
*C12P 7/10* (2006.01)   *C12N 1/36* (2006.01)
*C12N 9/92* (2006.01)   *C12N 9/12* (2006.01)
*C12N 15/81* (2006.01)

(86) Numéro de dépôt international:
**PCT/FR2015/050364**

(87) Numéro de publication internationale:
**WO 2015/121595 (20.08.2015 Gazette 2015/33)**

(54) **SOUCHE FERMENTANT LES PENTOSES A PROPAGATION OPTIMISÉE**

PENTOSE-PENTOSE-FERMENTIERENDER STAMM MIT OPTIMIERTER VERMEHRUNG

PENTOSE PENTOSE-FERMENTING STRAIN WITH OPTIMIZED PROPAGATION

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **17.02.2014 FR 1451246**

(43) Date de publication de la demande:
**28.12.2016 Bulletin 2016/52**

(83) **Déclaration conformément à la règle 32(1) CBE
(solution de l'expert)**

(73) Titulaire: **Lesaffre et Compagnie
75001 Paris (FR)**

(72) Inventeurs:
• **DESFOUGERES, Thomas**
**F-59960 Neuville En Ferrain (FR)**
• **PIGNEDE, Georges**
**F-59700 Marcq-en-Baroeul (FR)**
• **TECHEL, Jennifer**
**B-7783 Le Bizet (BE)**

(74) Mandataire: **Cabinet Laurent & Charras
Le Contemporain
50 Chemin de la Bruyère
69574 Dardilly Cedex (FR)**

(56) Documents cités:
**WO-A1-2005/121337   WO-A1-2011/079388
WO-A1-2012/072793   WO-A1-2013/081700**

**WO-A1-2013/178915   WO-A1-2013/178918**

• **SPENCER J F T ET AL: "Hybridization of
non-sporulating and weakly sporulating strains
of brewer's and distiller's yeasts", JOURNAL OF
THE INSTITUTE OF BREWING, INSTITUTE OF
BREWING. LONDON, GB, vol. 83, no. 5, 1977,
pages 287-289, XP008145123, ISSN: 0046-9750**
• **L. MORALES ET AL: "Evolutionary Role of
Interspecies Hybridization and Genetic
Exchanges in Yeasts", MICROBIOLOGY AND
MOLECULAR BIOLOGY REVIEWS, vol. 76, no. 4,
décembre 2012 (2012-12), pages 721-739,
XP055144370, ISSN: 1092-2172, DOI:
10.1128/MMBR.00022-12**
• **D. PINEL ET AL: "Saccharomyces cerevisiae
Genome Shuffling through Recursive Population
Mating Leads to Improved Tolerance to Spent
Sulfite Liquor", APPLIED AND ENVIRONMENTAL
MICROBIOLOGY, vol. 77, no. 14, 15 juillet 2011
(2011-07-15), pages 4736-4743, XP055144405,
ISSN: 0099-2240, DOI: 10.1128/AEM.02769-10**
• **MEKONNEN M DEMEKE ET AL: "Combining
inhibitor tolerance and D-xylose fermentation in
industrial Saccharomyces cerevisiae for efficient
lignocellulose-based bioethanol production",
BIOTECHNOLOGY FOR BIOFUELS, vol. 6, no. 1,
2013, page 120, XP055144407, ISSN: 1754-6834,
DOI: 10.1016/j.biortech.2004.12.034**

• WEI ZHANG ET AL: "Improved ethanol production by a xylose-fermenting recombinant yeast strain constructed through a modified genome shuffling method", BIOTECHNOLOGY FOR BIOFUELS, BIOMED CENTRAL LTD, GB, vol. 5, no. 1, 18 juillet 2012 (2012-07-18), page 46, XP021108102, ISSN: 1754-6834, DOI: 10.1186/1754-6834-5-46

LISTAGE DE SEQUENCES

<110> LESAFFRE ET COMPAGNIE

<120> Souches fermentant les pentoses à propagation optimisée

<130> DEM114FR

<160> 5

Sequence

<210> 1
<211> 23
<212> DNA
<213> Saccharomyces cerevisiae

<400> 1
agtcacatca agatcgttta tgg          23

Sequence

<210> 2
<211> 23
<213> Saccharomyces cerevisiae

<400> 2
gcacggaata tgggactact tcg        23

Sequence

<210> 3
<211> 23
<212> DNA
<213> Saccharomyces cerevisiae

<400> 3
actccacttc aagtaagagt ttg        23

Sequence

<210> 4
<211> 21
<212> DNA
<213> Saccharomyces cerevisiae

<400> 4
tagttgtcag tgcaatcctt c        21

Sequence

<210> 5
<211> 22
<212> DNA
<213> Saccharomyces cerevisiae

<400> 5
tatacacata tacagcatcg ga        22

**Description**

La présente demande appartient au domaine des souches de levures améliorées, plus particulièrement dans le but de produire des alcools, par exemple du bioéthanol. Le procédé selon l'invention fait appel à des techniques combinées de génétique et de biologie moléculaire pour générer des microorganismes recombinants.

Proposer des alternatives de biotechnologie blanche pour générer des biocarburants est un enjeu considérable au vu des coûts des énergies fossiles, des problèmes potentiels d'approvisionnement et des impacts sur l'environnement. La biomasse végétale est une matière première renouvelable de choix pour la production de biocarburants dans le cadre du développement durable.

Contexte

On entend par biomasse végétale toute biomasse qui peut soit correspondre à des excès ou des sous-produits de cultures agricoles et agroindustrielles telles que le maïs, la canne à sucre, le blé, le sorgho, les arbres de charpente et d'ameublement, soit correspondre à des cultures spécifiques telles que *Miscanthus gigantheus,* le panic érigé, les taillis à courte et très courte rotation. La biomasse végétale est un mélange de cellulose riche en hexoses, d'hemicellulose riche en pentoses et de lignine qui est un polymère de composés phénoliques hydrophobes. Classiquement trois étapes sont décrites pour la conversion de la biomasse végétale en bioéthanol. :

- Le prétraitement qui met en oeuvre des procédés mécaniques, chimiques et/ou de chauffage dans le but d'optimiser l'hydrolyse ultérieure de la biomasse ou d'une fraction de la biomasse;
- l'hydrolyse de la biomasse ou d'une fraction de la biomasse par action de réactifs, solvants ou d'enzymes avec les conditions de température et de pH appropriées. L'objectif de cette hydrolyse est la libération des principaux monosaccharides fermentescibles. Les enzymes utilisées doivent à la fois être stables et présenter une cinétique d'hydrolyse acceptable ;
- la fermentation des sucres fermentescibles qui doit être robuste, rapide, et utiliser l'ensemble des sucres disponibles, c'est-à-dire à la fois les sucres en C6 (hexoses) et les sucres en C5 (pentoses).

La première et la deuxième étape ou la deuxième et la troisième étape peuvent être combinées ou effectuées séparément.

Concrètement, l'étape de fermentation proprement dite est précédée d'une propagation du microorganisme qui effectuera la fermentation, et suivie d'une distillation. La propagation est en général effectuée dans la phase liquide issue de la saccharification enzymatique, par exemple séquentiellement dans deux cuves, la seconde étant significativement plus volumineuse que la première. Ces paramètres varient suivant les différents procédés industriels, le savoir-faire et les habitudes.

Améliorer l'ensemble du processus peut se faire en combinant les étapes et/ou en les améliorant séparément, par exemple en optimisant les enzymes utilisées pour l'hydrolyse ou les microorganismes responsables de la fermentation. Dans ce dernier cas, il est par exemple utile d'améliorer les aptitudes à transformer les sucres, plus particulièrement en permettant la transformation à la fois des hexoses et des pentoses, et/ou de limiter leur sensibilité aux inhibiteurs présents dans les hydrolysats lignocellulosiques ou dans les solutions de fermentation, tels l'acidité ou la température. Par exemple, *Saccharomyces cerevisiae,* qui est une levure couramment utilisée par les producteurs de bioethanol, n'est pas capable de métaboliser le D-xylose mais peut l'absorber *via* des transporteurs non spécifiques. Le transport est effectué par le biais des transporteurs d'hexoses qui présentent une affinité pour le D-xylose plus faible que pour le D-glucose. Ainsi, il peut être intéressant non seulement de manipuler génétiquement la cellule pour lui donner la capacité de métaboliser le D-xylose, mais également de la transformer pour qu'elle absorbe plus efficacement ledit sucre.

Pour une revue sur ces sujets, voir par exemple la Grange et al., 2010, Appl. Microbiol. Biotechnol., 87 : 1195-1208 ou Jordan et al., 2012, Biochem. J., 442 : 241-252.

La présente invention a trait à la transformation d'une cellule pour pallier des carences métaboliques, en lui conférant également la capacité à transformer le D-xylose et, avantageusement, à améliorer sa propriété d'absorption dudit sucre.

Etat de la technique

Dans le domaine de la transformation des levures, la demande de brevet WO 2010000464 A1 décrit des levures aptes à fermenter les sucres en C5, particulièrement le xylose, grâce à une transformation desdites levures par introduction d'un gène exogène codant une xylose isomérase fonctionnelle. En l'occurrence, la xylose isomérase est issue de *Clostridium phytofermentans.*

La demande de brevet WO 2012072793 A1 de la Demanderesse vise à optimiser le processus de transformation du xylose par une levure pour le rendre compatible avec une application industrielle. Dans ce but, ladite demande décrit l'introduction de cassettes d'expression d'un gène codant une enzyme apte à transformer tout hydrate de carbone (notamment D-xylose) en xylulose (D-xylulose) et d'un gène codant une enzyme apte à transformer tout pentol (notamment le xylitol) en xylulose en une seule étape. Ceci rend toute souche ainsi modifiée particulièrement performante pour la croissance sur et/ou pour la fermentation du xylose. Ce document décrit par exemple la souche de levure déposée à la CNCM (Collection Nationale de Cultures de Microorganismes, Institut Pasteur, 25 rue du Docteur Roux, 75724 Paris Cedex 15) le 5 octobre 2011 sous le numéro I-4538.

La demande de brevet WO 2013178915 A1, également déposée par la Demanderesse, améliore encore le procédé en transformant les levures afin de les rendre à la fois aptes à fermenter les pentoses et à résister à au moins un inhibiteur de fermentation. Ladite demande décrit l'obtention d'une levure hybride par un procédé combinant un aspect fonctionnel ciblé (transformation des pentoses) et un criblage sur l'aptitude des ségrégeants intermédiaires à résister à l'acide acétique. Dans ce cas, les cellules sélectionnées par le critère « résistance à l'acide acétique » sont peu diversifiées. Le document WO2011/079388 décrit une souche recombinante apte à fermenter le xylose mais ne contenant pas de gène codant une xylose isomérase. En outre, sa sélection est réalisée en milieu riche.

Enfin, la demande de brevet WO 2013178918 A1, également issue des travaux de la Demanderesse, vise à sécuriser le procédé d'obtention de souches de levure améliorées, *via* l'intégration d'au moins un gène d'intérêt dans une région génétiquement liée à un locus d'expression du type sexuel, puis le croisement avec des ségrégeants Mat a d'intérêt, c'est-à-dire susceptibles d'apporter une ou des caractéristiques phénotypiques d'intérêt que l'on souhaite voir cohabiter avec la modification génétique liée au locus sexuel. Ainsi, après sporulation de la souche de levure génétiquement modifiée, la ou les modifications génétiques ségrégent conjointement et se situent toutes dans un ségrégeant du type sexuel correspondant (Mat alpha). Concrètement, l'utilisation dudit procédé pour la construction d'un hybride permet d'être certain d'un caractère donné de cet hybride, par exemple du caractère « aptitude à métaboliser les pentoses ». Ce procédé original est d'autant plus intéressant qu'il est applicable à tout gène, pour tout type d'amélioration possible et à toute souche de levure qui a un cycle haplodiplophasique. Un cycle haplodiplophasique est un cycle de reproduction qui alterne une phase haploïde et une phase diploïde et au cours duquel l'organisme considéré peut se multiplier par mitose aussi bien à l'état haploïde qu'à l'état diploïde.

Suivant une démarche proche, Demeke et al., 2013, Biotechnology for Biofuels, 6 : 89 intègrent de façon stable dans le génome de la levure des cassettes d'expression de gènes permettant l'utilisation du D-xylose et du L-arabinose. Les hybrides obtenus sont également soumis à une mutagénèse, à un seul cycle de brassage, puis sélectionnés par leur aptitude à utiliser le D-xylose dans un milieu riche en inhibiteurs. Les auteurs soulèvent la question de la propagation et rapportent que la souche qu'ils ont obtenue présente un taux de propagation moyen. Ils suggèrent que les modifications génétiques à l'origine du taux de propagation plus lent seraient apparues durant la mutagénèse et le brassage. Ceci n'incite pas à utiliser la mutagénèse et/ou le brassage pour améliorer les aptitudes d'une souche à se multiplier.

Quoique destinés à améliorer des souches de levures industrielles, les travaux de l'art antérieur ne se soucient pas d'une étape cruciale pour le processus industriel de fabrication de biocarburant : la propagation. Egalement appelée multiplication, prolifération ou production de biomasse, la propagation est préalable à la phase de fermentation proprement dite. Le but est d'obtenir de façon systématique une quantité de biomasse optimale pour la fermentation. Elle est réalisée par l'industriel qui effectuera la fermentation sur un jus généré au cours du procédé, généralement sur le milieu complexe qui sera soumis à la fermentation. Le jus de propagation peut ainsi être un jus riche en pentoses, un jus riche en hexoses ou encore un mélange de pentoses et d'hexoses. Ce milieu de fermentation peut être légèrement dilué, enrichi en nutriments, aéré afin de permettre une croissance rapide et suffisante et permettre ainsi systématiquement une fermentation satisfaisante. L'apport des nutriments tels que une source d'azote et de phosphore minérales, tels que des minéraux sont ainsi généralement réalisés et en proportion plus importante qu'en fermentation. L'apport de composés tels que les vitamines et des composés organiques tels que des acides aminés ou des acides puriques ou pyrimidiques est évité car trop couteux. L'industriel compte généralement sur l'apport par le milieu proprement dit ou par des recyclages de milieu fermentés. Seul l'apport de vitamines peut être envisagé afin de sécuriser les apports par les milieux industriels qui peuvent être variables et entrainer une variabilité de la croissance ayant lieu.

Au-delà de la quantité de croissance obtenue au cours de la propagation, la rapidité de la croissance de la levure est également un point critique d'une propagation réussie. En effet, la vitesse de la croissance de la levure va limiter la durée de la propagation et permettre de limiter l'enrichissement relatif du milieu par des contaminants du milieu tels que les lactobacilles ou des levures sauvages. Une trop forte contamination de la propagation conduira à des pertes de rendement de production d'éthanol en fermentation. Même si des cytostatiques tels que le Lactrol® ou les extraits acides de houblon peuvent être utilisés pour limiter la croissance des bactéries, disposer d'une levure ayant une croissance rapide et importante reste un point clé de la réussite des propagations et des fermentations dans lesquelles les propagations sont transférées.

Ainsi disposer d'une levure ayant des besoins nutritionnels les plus réduits possibles qui ait une croissance rapide et importante en propagation est un point critique pour sécuriser l'ensemble du procédé industriel et permettre qu'il soit finalement rentable.

Suite à un retour d'expérience d'une médiocre capacité de propagation de l'une de ses souches, la Demanderesse a cherché à améliorer significativement la capacité d'une souche de levures à se propager, tout en restant stable génétiquement, et en conservant une bonne capacité à utiliser le D-xylose et à résister aux inhibiteurs généralement présents dans des « jus » lignocellulosiques. En d'autres termes, elle a cherché à réparer les carences métaboliques d'une souche.

A cet égard, la présente invention concerne un procédé d'obtention d'une souche de levures apte à se propager efficacement sur un milieu à faible potentiel nutritif et à résister aux inhibiteurs de la fermentation présents *ab initio* dans les hydrolysats ligno-cellulosiques ou formés au cours de la fermentation alcoolique, tout en gardant ses capacités à métaboliser les pentoses. Efficacement s'entend par comparaison entre au moins deux souches, une de référence étant jugée comme ne produisant pas assez de biomasse dans un temps donné compatible avec un processus industriel. Ledit procédé comprend les étapes suivantes :

a) croisement d'une souche de levure recombinante, apte à métaboliser les pentoses et à résister aux inhibiteurs de la fermentation, avec une souche de levure sauvage dépourvue de carences, apte à croitre dans un milieu minimum, la souche de levure recombinante comprenant au moins une copie d'un gène exogène de xylose isomérase et au moins une copie surnuméraire d'un gène de D-xylulokinase intégrées au génome dans une région génétiquement liée à un locus d'expression du type sexuel et ségrégeant conjointement,

b) au moins deux cycles de brassage génétique par sporulation et/ou hybridation aléatoires, sans sélection entre deux,

c) sélection de la population obtenue à l'étape b) selon un critère d'aptitude des souches à métaboliser le xylose, et

d) sélection de la population obtenue à l'étape c) selon un critère d'aptitude des souches à croitre dans un milieu à faible valeur nutritive étant un milieu pauvre en bases azotées comprenant du xylose comme seule source de carbone, une source d'azote minéral, une source de potassium, une source de phosphore, une source de soufre, une source de magnésium, une source de calcium, une source de fer, une source d'oligo-éléments et de l'eau,

sachant que les deux étapes de sélection peuvent être inversées.

Les ségrégeants intermédiaires obtenus après sporulation ne sont pas sélectionnés, par exemple sur leur capacité à résister aux inhibiteurs comme le suggère l'art antérieur, mais ils sont au contraire soumis directement à plusieurs étapes de brassage génétique sans sélection entre les brassages. Il est à noter que la capacité à métaboliser les pentoses, essentielle, est conservée grâce à l'insertion des gènes d'intérêt par une méthode selon la demande WO 2013/178918 A1. La sélection finale selon la présente invention est la levée des carences nutritionnelles à l'origine des médiocres performances de propagation.

L'invention porte également sur une souche obtenue selon le procédé, et déposée à la CNCM (Collection Nationale de Cultures de Microorganismes, Institut Pasteur, 25 rue du Docteur Roux, 75724 Paris Cedex 15) sous le numéro I-4749, le 16 mai 2013.

De façon avantageuse, une autre souche selon l'invention présente aussi une surexpression du gène *GAL2* permettant une meilleure entrée du xylose dans la cellule. La surexpression est rendue possible par l'addition d'une copie supplémentaire de ce gène, laquelle est de surcroit placée sous la dépendance du promoteur constitutif pADH1. Ce dernier contrôle habituellement l'expression du gène *ADH1.* La souche « fille » ainsi obtenue a été déposée à la CNCM (Collection Nationale de Cultures de Microorganismes, Institut Pasteur, 25 rue du Docteur Roux, 75724 Paris Cedex 15) sous le numéro I-4829 le 12 décembre 2013.

L'invention porte également sur l'utilisation d'une souche selon l'invention pour transformer des sucres, plus particulièrement des hexoses et des pentoses, en éthanol.

Pour s'assurer de la propagation efficace de la souche obtenue par le procédé selon l'invention, les inventeurs ont défini différents milieux « minimaux ».

Un milieu minimum est un milieu comprenant une source de carbone ($C_xH_yO_z$), une source d'azote minéral, une source de potassium, une source de phosphore, une source de soufre, une source de magnésium, une source de calcium, une source de fer, une source d'oligoéléments et de l'eau. C'est un milieu à faible valeur nutritive.

Le tableau I ci-dessous regroupe les différents milieux utilisés par les inventeurs : le milieu à faible potentiel nutritif, pauvre en bases azotées, utilisé dans le procédé selon l'invention est le milieu « Pref ». Ce milieu peut être un milieu minimal pour des souches selon l'invention, mais s'avérer être un milieu infra-minimal pour des souches qui ne répondent pas à l'invention.

Tableau I : Milieux de culture

|  | YFC (g/L) | YFX (g/L) | YFI1 (g/L) | Milieu Pref (g/L) |
|---|---|---|---|---|
| Eau distillée qsp | 1000 | 1000 | 1000 | 1000 |

(suite)

|  | YFC (g/L) | YFX (g/L) | YFI1 (g/L) | Milieu Pref (g/L) |
|---|---|---|---|---|
| Glucose | 58 | 0 | 150 | 0 |
| Xylose | 25 | 70 | 0 | 20 |
| EXL type J | 0 | 5 | 5 | 0 |
| $(NH_4) PO_4$ | 0 | 4,7 | 4,7 | 0 |
| $(NH_4)_2 SO_4$ | 0 | 0 | 0 | 5 |
| Hydrolysat de protéine | 0 | 0 | 0 | 3 |
| Urée | 3 | 0 | 0 | 0 |
| Acide Phosphorique | 0,85 | 0 | 0 | 0 |
| Acide citrique | 0 | 11,4 | 11,4 | 0 |
| Citrate trisodique | 0 | 13,5 | 13,5 | 0 |
| Acide acétique | 5 | 0 | 4 | 0 |
| $ZnSO_4$ | 0,021 | 0,021 | 0,021 | 0,04 |
| $MgSO_4 7H_2O$ | 1 | 1 | 1 | 0,5 |
| $KH_2PO_4$ | 0 | 0 | 0 | 1 |
| NaCl | 0 | 0 | 0 | 0,1 |
| $CaCl_2$ | 0 | 0 | 0 | 0,1 |
| $H_3BO_3$ | 0 | 0 | 0 | 0,005 |
| $CuSO_4 5 H_2O$ | 0 | 0 | 0 | 0,06 |
| KI | 0 | 0 | 0 | 0,001 |
| $MnSO_4 H_2O$ | 0 | 0 | 0 | 0,004 |
| $Na_2 MoO_4 2 H_2O$ | 0 | 0 | 0 | 0,002 |
| $FeCl_3$ | 0 | 0 | 0 | 0,0002 |
| Acide folique | 0 | 0 | 0 | 0 |
| Thiamine | 0,018 | 0,018 | 0,018 | 0,07 |
| Pyridoxine | 0,0053 | 0,0053 | 0,0053 | 0,002 |
| Biotine KOH | 0,0018 | 0,0018 | 0,0018 | 0,002 |
| Panthoténate | 0,0038 | 0,0038 | 0,0038 | 0,002 |
| Acide nicotinique | 0,016 | 0,016 | 0,016 | 0,05 |
| Mésoinositol | 0,05 | 0,05 | 0,05 | 0,2 |
| Riboflavine | 0,001 | 0,001 | 0,001 | 0,002 |
| Para aminobenzoate | 0,0012 | 0,0012 | 0,0012 | 0,002 |
| Mono-sorbate oléate | 1 | 1 | 1 | 0 |
| EXL = extrait de levure | | | | |

On mentionnera également :

- le milieu Pref complémenté avec 3 g/L d'hydrolysat d'ARN de *Candida utilis,* hydrolysé par ajout de RNase A à 30 μg/L, appelé Pref + bases azotées dans la suite du texte et dans les figures ;

- le milieu YFI2 contenant : extrait de levure 10 g/kg, BactoPeptone 10 g/kg, glucose 55g/L, xylose 45 g/L, acétate 4 g/L, ajusté à pH 4,4 ;

- le milieu YEG contenant : extrait de levure 10g/L, glucose 20 g/L, et agarose 20 g/L.

L'hypothèse est ensuite que la souche obtenue aura la capacité de se propager efficacement dans les milieux complexes des industriels qui l'utiliseront pour produire du bioéthanol.

Les définitions suivantes sont données afin de mieux comprendre l'invention.

L'expression « souche de levure » désigne une population relativement homogène de cellules de levure. Une souche de levure est obtenue à partir de l'isolement d'un clone. Un clone donne naissance à une population de cellules obtenue à partir d'une seule cellule de levure.

La ségrégation correspond à la situation lors de laquelle, à l'issue de la méiose, le niveau de ploïdie est réduit. Par extension, un ségrégeant est une cellule viable issue de la méïose d'une cellule de niveau de ploïdie supérieure à 1.

Par l'expression « souche de levure dérivée », on désigne une souche de levure dérivée par un ou plusieurs croisements et/ou par mutation et/ou par transformation génétique.

Une souche de levure dérivée par croisement peut être obtenue par croisement d'une souche de levure selon l'invention avec la même souche de levure, avec une autre souche de levure selon l'invention, ou avec une autre souche de levure quelconque (à condition qu'elle puisse croiser, ce qui sous-entend pour l'homme du métier qu'elle est homozygote pour le locus Mat et qu'elle ne présente pas de mutation dans les gènes *STE*).

Une souche de levure dérivée par mutation peut être une souche de levure ayant subi au moins une mutation spontanée dans son génome ou au moins une mutation induite par mutagenèse. La ou les mutations d'une souche dérivée peuvent être silencieuses ou non.

Par l'expression « mutagenèse », on désigne le processus d'apparition d'une mutation. Classiquement, deux méthodes sont possibles, la mutagénèse aléatoire et la mutagénèse insertionnelle ou dirigée. La première consiste en l'application d'un traitement physique (par exemple rayonnement UV) ou d'un traitement par des agents chimiques mutagènes, qui induiront de manière aléatoire des mutations dans le génome de l'organisme étudié. La seconde utilisera des méthodes de biologie moléculaire pour amener une modification précise (*i.e.* promoteur, gène, terminateur...), soit dans une région quelconque du génome, soit sur un locus précis. Par locus, on entend un emplacement physique précis et invariable sur un chromosome.

Une souche de levure dérivée par transformation génétique est une souche de levure dans laquelle a été introduite une séquence d'ADN qui est de préférence apportée par un plasmide ou intégrée directement dans le génome.

Par hexoses, on entend des sucres présentant 6 atomes de carbone, également appelés sucres en C6 ou plus simplement C6, utilisés comme source de carbone. Les représentants principaux des hexoses sous forme monomérique sont le glucose, le fructose et le galactose. Par analogie, les pentoses sont des sucres présentant 5 atomes de carbone, également appelés sucres en C5 ou C5. Les représentants monomériques principaux des pentoses sont le D-xylose et le L-arabinose .

Par propagation, on entend la multiplication, la prolifération ou la production de biomasse qui servira à ensemencer le milieu de fermentation. Elle peut être effectuée sur milieu naturel, issu par exemple de la transformation de biomasse végétale, ou sur milieu synthétique riche ou pauvre. D'une manière générale, la multiplication sera rapide sur un milieu riche et moins efficace sur un milieu pauvre, imposant à la cellule de fortes capacités métaboliques pour pallier la faible valeur nutritive (ou les carences) du milieu. L'hydrolysat lignocellulosique issu de la transformation de la biomasse végétale est un milieu complexe qui peut être un jus contenant essentiellement des pentoses, un jus qui contient essentiellement des hexoses ou un jus contenant un mélange d'hexoses et de pentoses. D'un point de vue industriel, un milieu synthétique « riche » serait assurément le choix le plus efficace pour la propagation mais ne serait pas viable économiquement. Un milieu minimal, c'est-à-dire comprenant le strict minimum, serait plus efficace mais définir sa composition n'est pas trivial. Pour ces raisons et pour des raisons de *continuum* du processus industriel, la propagation est le plus souvent réalisée sur la composition issue de l'étape précédente, autrement dit sur le jus ligno-cellulosique. Pour une revue, voir Bellissimi E, Richards C : Yeast propagation. In The alcohol textbook, a reference for the beverage, fuel and industrial alcohol industries. 5th édition. Edited by Ingledew WM, Kelsall DR, Austin GD, Kluhspies C. Nottingham : University Press; 2009:145-159.

Par carence métabolique d'une souche, on entend une défaillance d'une ou plusieurs voies métaboliques propre à générer des défauts de croissance ou de fermentation par la levure. Par auxotrophie, on entend une incapacité à produire un intermédiaire métabolique et essentiel pour le développement de la levure sur un milieu donné, c'est-à-dire un défaut métabolique qui fait que la souche ne poussera pas si tous les nutriments qui lui sont essentiels ne sont pas fournis de façon exogène.

Une souche de levure prototrophe est une souche capable de croître sur un milieu minimum. En particulier, une souche de levure prototrophe selon l'invention est capable de synthétiser toutes les bases azotées nécessaires à sa croissance.

Par inhibiteurs, on entend les inhibiteurs présents *ab initio* dans les hydrolysats ligno-cellulosiques ou formés au cours de la fermentation alcoolique, parmi lesquels on peut citer les produits phénoliques, le furfural et ses dérivés, l'hydroxy-méthyl furfural et ses dérivés, ou encore les acides faibles parmi lesquels l'acide acétique, l'acide formique ou l'acide lactique. Il est connu aussi que ces inhibiteurs sont néfastes pour les performances voire la survie de la levure. Alternativement, la pression osmotique, le pH (en particulier fortement acide), la température (supérieure à 35°C), ou l'éthanol produit peuvent également inhiber ou au moins limiter les capacités de fermentation d'une souche.

Une souche de levure apte à métaboliser le xylose est une souche de levure capable de convertir le xylose en éthanol, c'est-à-dire capable de fermenter le xylose.

La conversion du xylose en éthanol résulte de l'isomérisation directe ou indirecte du xylose en xylulose, suivie de l'utilisation du xylulose ainsi obtenu dans la partie non oxydative de la voie des pentoses phosphate.

Une souche de levure apte à métaboliser le xylose au sens de l'invention est une souche de levure qui convertit au moins 70%, de préférence au moins 80%, plus préférentiellement au moins 90 % du xylose en éthanol en 60 heures dans un milieu de fermentation comprenant 55 g de glucose et 45 g de xylose par kg de milieu de fermentation, en condition anaérobie.

L'ensemencement de la souche de levure utilisée pour mesurer le pourcentage de xylose converti en éthanol est de préférence 0,25 g en matière sèche / kg de milieu de fermentation.

La durée de 60 heures est calculée à partir de l'ensemencement de la souche de levure dans le milieu de fermentation.

Le milieu de fermentation utilisé pour mesurer le pourcentage de xylose converti en éthanol est de préférence un milieu synthétique.

Un milieu synthétique est un milieu dont la composition chimique exacte est connue.

La fermentation est conduite de préférence à 32°C, sous agitation modérée, par exemple 90 rpm.

L'agitation est modérée pour ne pas être oxygénante.

Le pH du milieu est de préférence contrôlé, par exemple par le pouvoir tampon d'un couple acide/base, par exemple le pouvoir tampon acide acétique/acétate dans le milieu YFGX.

La quantité d'éthanol présente dans le milieu de fermentation est mesurée par tout moyen approprié connu de l'homme du métier.

Il peut s'agir d'une mesure directe de l'éthanol produit ou d'une mesure indirecte via un paramètre corrélé à la production d'éthanol, tel que la perte de masse.

Par exemple, la production d'alcool peut être mesurée par chromatographie, notamment par HPLC (High Performance Liquid Chromatography), un kit enzymatique (par exemple le kit de dosage de l'éthanol de Boehringer), ou un dosage par le dichromate de potassium.

La quantité de xylose présente dans le milieu de fermentation est mesurée par tout moyen approprié connu de l'homme du métier, de préférence par chromatographie, notamment par HPLC.

L'utilisation d'un milieu de fermentation comprenant à la fois du glucose et du xylose permet d'évaluer la conversion du xylose en éthanol à partir d'une quantité de biomasse comparable pour les différentes souches de levure évaluées. En effet, les souches de levure fermentent en premier le glucose du mélange glucose et xylose, puis le glucose et le xylose, puis le xylose.

La capacité à métaboliser le xylose en présence d'au moins un inhibiteur de fermentation est qualifiée de résistance au dit inhibiteur de fermentation.

Brève description des figures

La Figure 1 illustre la propagation respective des souches I-4538 (souche recombinante parente, déposée à la CNCM le 5 Octobre 2011), EGAc1 déposée à la CNCM le 13 Mars 2014 sous la référence I-4839 (souche sauvage parente) et I-4749 (souche selon l'invention, déposée à la CNCM le 16 Mai 2013) sur les milieux à faible valeur nutritive Pref et Pref + bases azotées.

La Figure 2 illustre la comparaison entre la propagation de la souche I-4749 selon l'invention et la propagation de la souche parente I-4538 sur un milieu synthétique mimant un milieu de propagation industriel.

La Figure 3 illustre la production d'éthanol par la souche sauvage EGAc1 (I-4839), la souche recombinante parente I-4538 et la souche selon l'invention I-4749.

Description détaillée de l'invention

Initialement, il est apparu que la souche I-4538 présentait des carences métaboliques qui impactaient négativement l'efficacité de sa propagation sur des milieux complexes. Ladite souche I-4538 est l'une des souches obtenues selon la demande de brevet WO 2012072793 A1, comprenant au moins une copie d'un gène exogène codant une xylose isomérase, et une copie d'un gène exogène codant une xylitol déshydrogénase . Elle comprend également au moins une copie supplémentaire du gène XKS1 et des gènes de la voie des pentoses-phosphates. Concrètement, cette souche présente une bonne capacité à métaboliser le xylose et résiste aux inhibiteurs de fermentation issus de l'hydrolyse de la biomasse tels que les produits phénoliques, le furfural ou l'acide acétique. Ainsi, la phase de propagation sur un milieu pauvre en bases azotées contenant en outre du xylose a été analysée pour être ensuite améliorée. Il est apparu que de l'ARN hydrolysé autrement dit des bases azotées, étaient très favorable à la croissance de la souche I-4538. De façon intéressante, cette auxotrophie n'est pas présente sur un milieu contenant du glucose, ce qui indique que les voies de biosynthèse sont fonctionnelles, mais mal régulées dans un milieu contenant du xylose. La levure n'étant pas naturellement apte à métaboliser ce sucre, il est possible que l'expression des gènes requis pour la synthèse des bases azotées ne soit pas suffisante. De surcroit, les différentes étapes de transformations génétiques et d'irradiations aux UV ayant conduit à l'obtention de la souche sont probablement responsables de ces carences.

Dans une première étape, la souche I-4538 est hybridée avec une souche sauvage ne présentant pas de carence (souche référencée EGAc1 déposée à la CNCM le 13 Mars 2014 sous la référence I-4839 dans les exemples et les figures). Souche sauvage s'entend comme une souche non génétiquement modifiée. Cette étape a conduit à un hybride dont les carences métaboliques étaient en partie réparées mais ayant perdu une part significative de la capacité à fermenter rapidement le xylose.

L'étape d'hybridation est réalisée selon les techniques classiques, comme celles enseignées dans le chapitre 7 « Sporulation and Hydridization of Yeast » par R.R. Fowell, de l'ouvrage de référence « The Yeasts », Volume 1, édité par A.H. Rose et J. S. Harrison, 1969-Academic Press.

Une deuxième étape consiste en une recombinaison aléatoire du génome, plus précisément en quatre cycles de brassage du génome. Les cycles sont enchainés sans étape de sélection entre deux. Cette étape est réalisée selon une méthode adaptée de Hou, 2009, Biotechnol. Lett., 31 :671-677.

La population ainsi obtenue est sélectionnée selon un critère d'aptitude à métaboliser le xylose, puis selon un critère d'aptitude à se multiplier sur milieu à faible valeur nutritive, plus particulièrement sur l'aptitude à se passer de bases azotées dans le milieu de propagation.

Les deux critères de sélection peuvent être intervertis. Autrement dit, il est possible de sélectionner dans un premier temps sur la capacité à se multiplier en milieu carencé puis sur la capacité à fermenter le xylose ou d'abord sur la capacité à fermenter le xylose puis sur la capacité à se multiplier en milieu carencé.

La souche ainsi obtenue a été déposée à la CNCM (Collection Nationale de Cultures de Microorganismes, Institut Pasteur, 25 rue du Docteur Roux, 75724 Paris Cedex 15) sous le numéro I-4749.

De façon intéressante, la levée de l'auxotrophie vis-à-vis des bases azotées sur un milieu contenant du xylose comme seule source de carbone est transmissible aux souches qui descendent en droite ligne de la souche I-4749. Ainsi, différentes souches parmi lesquelles la souche déposée I-4829 ont été obtenues à partir de la souche I-4749. En effet, il a été observé que la protéine Gal2p est un transporteur d'hexoses également capable de transporter le xylose (Hamacher et al., 2002, Microbiology, 148 : 2783-2788). Ainsi, il est intéressant d'améliorer la capture du xylose par une souche de levure, par exemple *Saccharomyces cerevisiae,* que l'on aurait rendue capable de fermenter de xylose. Pour cette raison, une copie du gène *GAL2,* placée sous la dépendance d'un promoteur fort et constitutif (pADH1), a été introduite dans le génome de la souche. Il code pour un canal qui favorise l'entrée du xylose dans les cellules. Cette souche étant une souche de levure selon l'invention. La souche obtenue par cette transformation génétique supplémentaire a été déposée à la CNCM (Collection Nationale de Cultures de Microorganismes, Institut Pasteur, 25 rue du Docteur Roux, 75724 Paris Cedex 15) sous la référence I-4829.

Les levures sont obtenues par culture d'une souche de levure selon l'invention ou d'une souche de levure dérivée selon l'invention, notamment comme décrit dans le livre de référence « Yeast Technology », 2ème édition, 1991, G. Reed et T.W. Nagodawithana, publié par Van Nostrand Reinhold, ISBN 0-442-31892-8.

La multiplication des levures, à l'échelle industrielle, comprend en général au moins les deux premières étapes de l'ensemble des étapes suivantes :

- multiplication d'une souche de levure en plusieurs stades, d'abord en semi-anaérobiose, puis en aérobiose,

- séparation par centrifugation de la levure ainsi produite de son milieu de culture, pour obtenir une crème de levure liquide contenant environ entre 12 et 25% de matière sèche, voire une quantité plus élevée de matière sèche si la crème de levure est mélangée avec des produits osmolytes,

- filtration de la crème de levure liquide ainsi obtenue, en général sur un filtre rotatif sous vide, pour obtenir une levure fraîche déshydratée contenant de 26% à 35% de matière sèche,

- malaxage de ladite levure fraîche déshydratée, pour obtenir une masse homogène,

- extrusion de la levure ainsi obtenue, pour obtenir :

  o une levure pressée sous forme de pains de levure fraîche ou de levure fraîche émiettée, contenant environ 30% de matière sèche, ou

  o une levure sous forme de particules, en général de granules, si la levure est destinée à être séchée,

- éventuellement, séchage de manière ménagée, dans un courant d'air chaud, par exemple par fluidisation, des particules de levures obtenues par extrusion pour obtenir de la levure sèche.

L'étape de séchage est de préférence un séchage rapide ménageant en présence d'un émulsifiant.

Parmi les émulsifiants pouvant être utilisés lors de l'étape de séchage, on peut choisir le monostéarate de sorbitan, utilisé par exemple à une concentration d'environ 1,0% (en poids sur le poids de levure sèche).

Les levures selon l'invention peuvent être utilisées sous toute forme possible.

Par exemple, la présente invention a pour objet une levure telle que définie ci-dessus, caractérisée en ce qu'elle est sous forme de crème de levure, de levure pressée, de levure sèche ou de levure surgelée.

La présente invention a également pour objet un procédé de production d'au moins un produit de fermentation comprenant une étape de fermentation, en condition anaérobie ou semi-anaérobie, par une levure telle que définie ci-dessus dans un milieu de fermentation.

Le produit de fermentation est notamment choisi parmi l'éthanol, un métabolite obtenu à partir de l'éthanol ou un métabolite secondaire.

Un produit de fermentation préféré selon l'invention est l'éthanol.

La production d'éthanol est obtenue par fermentation alcoolique.

L'homme du métier sait déterminer les conditions appropriées pour une fermentation alcoolique.

A titre d'exemple, on peut se référer aux conditions de fermentation alcoolique décrites dans le livre de référence « Yeast Technology », 2ème édition, 1991, G. Reed et T.W. Nagodawithana, publié par Van Nostrand Reinhold, ISBN 0-442-31892-8.

Le milieu de fermentation comprend les éléments suivants : au moins une source de carbone fermentescible, au moins une source d'azote, au moins une source de soufre, au moins une source de phosphore, au moins une source de vitamines et/ou au moins une source de minéraux.

La source de carbone est par exemple apportée sous la forme d'un sucre immédiatement assimilable par la levure, un pentose tel que le xylose, du glycérol, de l'éthanol et/ou un mélange de ceux-ci.

Un sucre immédiatement assimilable par la levure est par exemple un sucre simple de type glucose, fructose ou galactose, un disaccharide de type saccharose et/ou un mélange de ces sucres.

La source de carbone peut être apportée sous la forme d'un sirop de glucose, d'un sirop de fructose, d'un sirop de saccharose, de mélasses, d'EP2 (Egout Pauvre issu de la 2ème cristallisation du sucre), d'un hydrolysat de tout ou partie d'un matériel végétal et/ou d'un mélange de ceux-ci.

La source d'azote est par exemple apportée sous la forme de sulfate d'ammonium, hydroxyde d'ammonium, di-ammonium phosphate, ammoniac, urée et/ou une combinaison de ceux-ci.

La source de soufre est par exemple apportée sous la forme de sulfate d'ammonium, sulfate de magnésium, acide sulfurique et/ou une combinaison de ceux-ci.

La source de phosphore est par exemple apportée sous la forme d'acide phosphorique, phosphate de potassium, di-ammonium phosphate, mono-ammonium phosphate, et/ou une combinaison de ceux-ci.

La source de vitamines est par exemple apportée sous la forme de mélasse, hydrolysat de levure, solution de vitamine pure ou d'un mélange de vitamines pures et/ou une combinaison de ceux-ci.

La source de vitamines apporte à la levure l'ensemble des vitamines dans des quantités au moins équivalentes à celles recommandées dans les ouvrages de référence. Plusieurs sources de vitamines peuvent être associées.

La source de minéraux est par exemple apportée sous la forme de mélasse, un mélange de sels minéraux et/ou leur combinaison.

La source de minéraux apporte à la levure l'ensemble des macroéléments et oligoéléments dans des quantités au moins équivalentes à celles recommandées dans les ouvrages de référence. Plusieurs sources de minéraux peuvent être associées.

Une même substance peut apporter plusieurs éléments différents.

La présente invention a particulièrement pour objet un procédé tel que défini ci-dessus de production d'au moins un produit de fermentation, de préférence l'éthanol, comprenant une étape de fermentation, en condition anaérobie ou semi-anaérobie, par une levure telle que définie ci-dessus dans un milieu de fermentation comprenant du xylose et/ou au moins un inhibiteur de fermentation. Préférentiellement, le milieu de fermentation comprend au moins un hydrolysat de tout ou partie d'un matériel végétal.

Un hydrolysat de tout ou partie d'un matériel végétal peut être obtenu par une étape de prétraitement du matériel végétal, par exemple à température élevée et en présence d'acides ou de solvants organiques, éventuellement suivie d'une hydrolyse partielle ou totale des polymères de sucre, par voie enzymatique et/ou chimique et/ou thermique.

L'hydrolysat de tout ou partie d'un matériel végétal comprend donc un mélange de sucres provenant de l'hydrolyse des polymères de sucres, tels que la cellulose, l'hémicellulose et l'amidon.

L'inhibiteur de fermentation est par exemple choisi parmi un acide organique, le furfural, le HMF (hydroxyméthyl-furfural), un ou plusieurs composés phénoliques, la pression osmotique.

L'acide organique est par exemple choisi parmi l'acide acétique, l'acide lactique, l'acide formique, l'acide lévulinique.

La présente invention a également pour objet l'utilisation d'une levure telle que définie ci-dessus pour la production d'au moins un produit de fermentation, de préférence dans un milieu de fermentation comprenant du xylose et au moins un inhibiteur de fermentation. Autrement dit, l'utilisation d'une levure selon l'invention permet la conversion et la métabolisation d'un matériel d'origine végétale comprenant du xylose.

Le produit de fermentation est tel que défini ci-dessus.

De préférence, le produit de fermentation est l'éthanol.

Le milieu de fermentation est tel que défini ci-dessus.

Les exemples ci-après sont destinés à mieux comprendre l'invention mais n'ont aucun caractère limitant.

EXEMPLES

**Croisement d'une souche recombinante avec une souche sauvage puis sporulation.**

La souche recombinante déposée à la CNCM (Collection Nationale de Cultures de Microorganismes, Institut Pasteur, 25 rue du Docteur Roux, 75724 Paris Cedex 15) sous le numéro I-4538 a été croisée avec une souche sauvage, en l'occurrence la souche de la Demanderesse EGAc1 (I-4839). Cette étape a été réalisée selon les techniques classiques, comme celles enseignées dans le chapitre 7 « Sporulation and Hydridization of Yeast » par R.R. Fowell, de l'ouvrage de référence « The Yeasts », Volume 1, édité par A.H. Rose et J. S. Harrison, 1969- Academic Press.

La souche EGAc2 déposée à la CNCM sous la référence I-4840 le 13 Mars 2014 a ainsi été obtenue. Cette souche présentait de bonnes performances de métabolisation du xylose et des carences réparées en partie seulement. En effet, la propagation restait de mauvaise qualité sur un milieu à faible valeur nutritive comprenant essentiellement du xylose et un hydrolysat de protéines.

La sporulation a été réalisée dans des milieux liquides dépourvus de source d'azote et contenant une source de carbone non fermentescible, de préférence un sel d'acétate, de préférence l'acétate de potassium.

**Mise en place d'une méthode rapide d'enrichissement en ségrégeants**

Dans la mesure où il ne semble pas possible d'obtenir 100% de ségrégeants, il était nécessaire d'éliminer les diploïdes qui n'avaient pas accompli de méïose.

Pour cela, la méthode de la dissection d'asques est usuellement mise en oeuvre mais présente l'inconvénient d'être très consommatrice de temps. Ainsi, la Demanderesse a utilisé une particularité des spores, à savoir qu'elles sont réputées pour leur plus grande résistance vis-à-vis de la température (Williams, 1936, J. Bacteriol., 32 (6) : 589-597), de l'absence de certains nutriments (Ho et Miller, 1978, Can. J. Microbiol., 24 (3) : 312-320) ou de certains solvants organiques (Dawes et Hardie, 1974, Mol. Gen. Genet., 131 (4) : 281-289).

Dans le cas présent, une méthode basée sur l'enrichissement à l'éther décrit par Dawes et Hardie (*supra*) a été utilisée. En effet, cette approche est simple et efficace.

En effet, de par la formation des ségrégeants dans la membrane du diploïde, ceux-ci ne sont pas entourés d'une bicouche phospholipidique mais de deux. En revanche, les diploïdes n'en ont qu'une seule. L'éther, lorsqu'il est employé avec un temps de contact optimal (variable d'une souche à l'autre) va lyser les membranes des diploïdes. L'intérêt de l'éther est donc double. D'une part, il tue les diploïdes sans affecter les ségrégeants si le temps de contact n'est pas trop long. D'autre part, il dégrade la bicouche phospholipidique de l'asque qui retient les ségrégeants sous la forme de tétrades, ce qui a pour conséquence de les libérer et de leur permettre de germer.

Cette méthode a été adaptée aux souches industrielles de la Demanderesse. Le temps de contact entre la suspension de levure et l'éther doit alors être étroitement contrôlé. Pour ce faire, 2 mL d'éther sont mis en contact avec 2 mL de la suspension de levure contenant environ $2 \times 10^7$ structures de sporulation. Cette dernière est obtenue après 5 jours en conditions de sporulation. L'ensemble est ensuite agité au vortex durant un temps de contact total variant de 30 secondes à 2 minutes.

Une aliquote contenant 1000 cellules / mL est immédiatement étalée à hauteur de 100 μL / boite sur un milieu contenant :
Extrait de levure 5 g/L, glucose, 20 g/L, agar 30 g/L, eau qsp 1 L.. Après 48 heures de croissance, les colonies sont utilisées pour faire des « PCR sur colonie » en utilisant les amorces SEQ ID N°1, SEQ ID N°2 et SEQ ID N°3 correspondant respectivement aux amorces Mat1, Mat2 et Mat3 telles que décrites dans la demande WO2013/178918 A1. L'objectif de cette analyse PCR est de différencier les souches haploïdes des souches diploïdes.
Les différents essais de temps de contact réalisés montrent que, dans le cas de la souche EGAc2 (I-4840), une exposition de 1 minute est suffisante pour enrichir à plus de 98 % en souches haploïdes.

**Mise en place du protocole d'hybridation en masse**

De nouveaux hybrides ont été générés à partir des suspensions enrichies en ségrégeants en réalisant une phase d'hybridation en masse. Pour cela, 1 mL de la suspension de ségrégeants a été inoculé dans 50 mL de milieu YPG contenant extrait de levure 10 g/L, Bactopeptone 20 g/L, glucose 20 g/L et eau déminéralisée qsp 1 L. Après 16 heures dans ce milieu, une observation microscopique permet de vérifier la formation de zygotes. Afin de favoriser le développement de ces hybrides, toutes les 24 heures, 200 μL de la culture sont inoculés dans 50 mL de milieu YEG frais. Après 5 jours de repiquage, les nouveaux hybrides peuvent être réintroduits dans un cycle de sporulation en masse.
Afin de s'assurer de l'efficience de cette étape d'hybridation en masse, 100 cellules ont été étalées par boite de Petri contenant du YEG. Le signe sexuel des cellules constituant 139 des colonies formées a ensuite analysé par PCR sur ADNg. Ces colonies ont été choisies de manière aléatoire. Les PCR réalisées en utilisant les amorces SEQ ID N°1, SEQ ID N°2 et SEQ ID N°3 (*supra*) ont montré que seules 2 colonies sur les 139 testées étaient haploïdes.

**Validation du brassage génétique dans la population d'hybrides obtenue**

Analyse du génotype de l'hybride de départ

Une des méthodes pour valider la qualité du brassage génétique a consisté à étudier la répartition des allèles de deux loci liés au gène *GRE3*. En effet, la délétion des deux copies du gène *GRE3* correspondant à un génotype « gre3 null » a été réalisée dans les souches C5 ayant conduit à la souche I-4538. Cette disruption a été transmise à la souche EGAc2 (I-4840) qui, du fait de la non-délétion de *GRE3* dans EGAc1 (I-4839), est donc hétérozygote.
L'éventuelle transmission de ce caractère dans les ségrégeants issus de la souche EGAc2 (I-4840) a été recherchée. Pour cela, des PCR ont été effectuées avec une amorce propre au promoteur pGRE3 TAGTTGTCAGTGCAATCCTTC (SEQ ID N°4) et une seconde amorce propre au terminateur tGRE3 TATACACATATACAGCATCGGA (SEQ ID N°5) de *GRE3*. Les résultats obtenus ont montré qu'il était possible de différencier les copies sauvages du gène *GRE3* qui donnent un fragment de 1200 pb, des formes délétées qui donnent un fragment de 200 pb.
Ainsi certains ségrégeants de la souche EGAc2 (I-4840) présentaient exclusivement la version délétée alors que d'autres présentaient seulement la copie sauvage de *GRE3*. De manière plus surprenante, une troisième catégorie de ségrégeants présentaient 2 copies du gène *GRE3,* l'une sauvage et l'autre délétée. Ce résultat s'explique par le fait qu'il n'y a pas deux, mais quatre copies du gène *GRE3* dans la souche EGAc1 (I-4839). Dans ce cas, il y a deux copies de *GRE3* dans le ségrégeant de EGAc1 (I-4839) qui a donné EGAc2 (I-4840). Ainsi le génotype de la souche EGAc2 (I-4840) est le suivant :

$$\frac{GRE3}{gre3\Delta} ; \frac{GRE3}{-}$$

Un tel génotype est dû à la présence de *GRE3* à deux loci différents dans la souche EGAc1 (I-4839) contre 1 seul locus dans la souche I-4538. De surcroit, dans la souche I-4538, le gène *GRE3* a été délété. Ainsi, parmi 137 souches diploïdes obtenues à l'issue d'un premier cycle de brassage génétique de la souche EGAc2 (I-4840), les gènes *GRE3* et la dispersion des allèles entre les hybrides ont été étudiés.

Détermination des ségrégeants attendus

En regard du génotype *GRE3*, un hybride comme EGAc2 (I-4840) peut donc donner 4 types de ségrégeants. Ces derniers sont répertoriés dans le tableau II ci-dessous. Les ségrégeants de génotype gre3Δ ; - et *GRE3* ; *GRE3* sont dits de génotypes parentaux car tous leurs allèles proviennent d'un seul parent. Par opposition, les ségrégeants *GRE3* ; - et gre3Δ ; *GRE3* sont dits recombinants. Pour chaque cas, il existe une forme Mat a et une forme Mat alpha.

Tableau II : Génotype des différents ségrégeants pouvant être obtenus

| Hybride de départ | Ségrégeants | |
|---|---|---|
| | *Mat a* | *Mat α* |
| | *GRE3 ; GRE3* | *gre3Δ ; -* |
| $\dfrac{GRE3}{gre3\Delta} ; \dfrac{GRE3}{-}$ | *GRE3 ; -* | *gre3Δ ; GRE3* |
| | *gre3Δ ; GRE3* | *GRE3 ; -* |
| | *gre3Δ ; -* | *GRE3 ; GRE3* |

Une manière d'analyser la qualité du brassage génétique a consisté à rechercher la répartition des allèles issus de l'hybride parental dans les ségrégeants. Ainsi, dans l'hypothèse où les loci sont indépendants, la probabilité d'obtenir un ségrégeant parental est égale à celle d'obtenir un ségrégeant recombinant.

Toutefois, l'approche visait à travailler sans sélectionner les ségrégeants. En effet, le but était de déterminer les spores qui avaient effectivement été impliquées dans la formation des hybrides. C'est donc à partir de ces derniers que l'analyse génétique a été effectuée.

Analyse des hybrides obtenus

Les hybrides susceptible d'être obtenus à l'issue de ce brassage génétique sont indiqués dans le tableau III. Les génotypes de chaque type d'hybride sont référencés dans la case correspondante. En regard des profils PCR avec les amorces pGRE3 (SEQ ID N°4) et tGRE3 (SEQ ID N°5), ces génotypes se répartissent en 3 groupes :

Groupe 1 : Une bande unique à une taille de 200 pb (cellule en blanc dans le tableau)

Groupe 2 : Deux bandes de 200 pb et de 1200 pb (cellules en gris clair dans le tableau)

Groupe 3 : Une bande unique de 1200 pb (cellules en gris sombre dans le tableau)

Tableau III : Génotype des différents hybrides pouvant être obtenus. Ligne 2 et colonne 2, il s'agit des ségrégeants, les autres cases concernent les hybrides.

| | | Mat α | | | |
|---|---|---|---|---|---|
| | | gre3Δ ; - | gre3Δ ; GRE3 | GRE3 ; - | GRE3 ; GRE3 |
| Mat a | gre3Δ ; - | $\frac{gre3\Delta}{gre3\Delta} ; \frac{-}{-}$ | $\frac{gre3\Delta}{gre3\Delta} ; \frac{GRE3}{-}$ | $\frac{GRE3}{gre3\Delta} ; \frac{-}{-}$ | $\frac{GRE3}{gre3\Delta} ; \frac{GRE3}{-}$ |
| | gre3Δ ; GRE3 | $\frac{gre3\Delta}{gre3\Delta} ; \frac{-}{GRE3}$ | $\frac{gre3\Delta}{gre3\Delta} ; \frac{GRE3}{GRE3}$ | $\frac{GRE3}{gre3\Delta} ; \frac{-}{GRE3}$ | $\frac{GRE3}{gre3\Delta} ; \frac{GRE3}{GRE3}$ |
| | GRE3 ; - | $\frac{gre3\Delta}{GRE3} ; \frac{-}{-}$ | $\frac{gre3\Delta}{GRE3} ; \frac{GRE3}{-}$ | $\frac{GRE3}{GRE3} ; \frac{-}{-}$ | $\frac{GRE3}{GRE3} ; \frac{GRE3}{-}$ |
| | GRE3 ; GRE3 | $\frac{gre3\Delta}{GRE3} ; \frac{-}{GRE3}$ | $\frac{gre3\Delta}{GRE3} ; \frac{GRE3}{GRE3}$ | $\frac{GRE3}{GRE3} ; \frac{-}{GRE3}$ | $\frac{GRE3}{GRE3} ; \frac{GRE3}{GRE3}$ |

Les hybrides du groupe 1 peuvent être facilement identifiés car ils présentent un profil PCR comparable à celui de la souche I-4538 lorsque les amorces pGRE3 et tGRE3 sont utilisées. Il est à noter que tous les hybrides ayant ce génotype sont issus des ségrégeants Mat a ; gre3Δ ; - et Mat alpha ; gre3Δ ; -. Dans l'hypothèse d'une indépendance génétique, ces hybrides devraient représenter 1/16 de la population soit 6,25 %. Afin de vérifier cette hypothèse, les génotypes de 137 hybrides précédemment évoqués ont été analysés par PCR. Les résultats figurent dans le tableau IV ci-dessous.

Tableau IV : Résultats de l'analyse par PCR du génotype des hybrides obtenus

| Groupe génotypique (PCR) | Nombre d'hybrides | Proportion de la population | Proportion attendue si les 2 loci GRE3 sont indépendants |
|---|---|---|---|
| Groupe 1 1 bande 200 pb | 16 | 11,6 % | 6,25 % (1/16) |
| Groupe 2 2 bandes 200 et 1200 pb | 89 | 64,5 % | 68,8 % (11/16) |
| Groupe 3 1 bande 1200 pb | 32 | 23,3 % | 25 % (4/16) |

La sur-représentation des hybrides du groupe 1 (11,6 % au lieu de 6,25 %) montre que les deux loci de GRE3 ne sont pas indépendants. En d'autres termes, cela signifie que lors de la méïose, la probabilité d'avoir un ségrégeant de type parental est plus importante que celle d'avoir un ségrégeant de type recombinant. Ce résultat permet de surcroît de déterminer la distance génétique en cM. Cette distance génétique est obtenue via l'équation suivante :

$$\text{Distance génétique (cM)} = \left( \frac{\textit{nombre de ségrégeants recombinants}}{\textit{nombre de ségrégeants totaux}} \right) X\ 100$$

Dans la présente analyse, le nombre total de ségrégeants est le nombre d'hybrides multiplié par 2 (soit 274).

Le nombre de ségrégeants recombinants est obtenu en soustrayant le nombre de ségrégeants parentaux au nombre total de ségrégeants. Or, comme indiqué plus haut, les hybrides du groupe 1 sont constitués exclusivement de ségrégeants parentaux. Cela implique que la probabilité d'avoir des ségrégeants parentaux gre3Δ ; - est égale à la racine carrée de la probabilité d'avoir un hybride du groupe 1. De plus, les ségrégeants parentaux sont équiprobables lors de la méïose. Le nombre de ségrégeants recombinants peut donc être déterminé par l'équation suivante :

$$nombre\ de\ ségrégeants\ recombinants$$
$$= nombre\ de\ ségrégeants\ total - nombre\ de\ ségrégeants\ parentaux$$

Avec :

$$nombre\ de\ ségrégeants\ parentaux =$$
$$nombre\ total\ d'hybrides\ X\ 4\ X\ \sqrt{\frac{nombre\ hybrides\ du\ groupe\ 1}{nombre\ d'hybrides\ total}}$$

Ainsi, le nombre de ségrégeants parentaux est de 186 et donc le nombre de ségrégeants recombinants est de 88. Ce résultat implique que la distance génétique entre les deux loci serait de 32 cM. Ce calcul de distance génétique permet donc de mesurer la probabilité d'avoir chaque type de ségrégeant.

Estimation du nombre de cellules de départ retrouvées dans la population finale

Un point important par rapport à la mesure de la qualité de la diversification de la population est la détermination du nombre d'hybrides de départ qui ont survécu à l'intégralité du traitement pour rester dans la population finale. Il est possible d'en estimer le nombre en s'appuyant sur les résultats de la détermination du génotype *GRE3*. En effet, les cellules dont le profil PCR présente deux bandes (à 200 pb et à 1200 pb) sont soit des hybrides obtenus par le brassage génétique, soit les cellules de départ qui n'ont pas été tuées lors de l'enrichissement à l'éther.

Souches du groupe 2=hybrides vrais du groupe 2+cellules de départ ayant survécu

Les résultats obtenus au paragraphe précédent permettent de déterminer à la fois le nombre d'hybrides vrais du groupe 2 et le nombre de souches du groupe 2.

Afin de déterminer le nombre d'hybrides vrais du groupe 2, il faut additionner la proportion de chaque type d'hydride qui compose ce groupe. Le tableau V ci-dessous recense les différents hybrides, ainsi que la probabilité de les obtenir. La probabilité d'obtenir un type d'hybride repose sur le produit de la probabilité d'obtention des deux ségrégeants qui le composent. Il est indiqué dans le paragraphe précédent que les ségrégeants parentaux représentent 68 % des ségrégeants (soit 34 % pour chaque ségrégeant parental). Par voie de conséquence, les ségrégeants recombinants représentent 32 % de tous les ségrégeants (soit 16 % pour chaque ségrégeant recombinant).

Tableau V : Probabilité d'obtenir chaque type d'hybride. Le code couleur pour les hybrides du groupe 1, et des groupes 2 et 3 respectivement est identique aux Tableau III et IV.

| | | | Mat α | | | |
|---|---|---|---|---|---|---|
| | | | Parental | Recombinant | Recombinant | Parental |
| | | | gre3Δ ; -<br>34 % | gre3Δ ; GRE3<br>16 % | GRE3 ; -<br>16 % | GRE3 ; GRE3<br>34 % |
| Mat a | Parental | gre3Δ ; -<br>34 % | 11,6 % | 5,4 % | 5,4 % | 11,6 % |
| | Recombinant | gre3Δ ;<br>GRE3<br>16 % | 5,4 % | 2,6 % | 2,6 % | 5,4 % |
| | Recombinant | GRE3 ; -<br>16 % | 5,4 % | 2,6 % | 2,6 % | 5,4 % |
| | Parental | GRE3 ;<br>GRE3<br>34 % | 11,6 % | 5,4 % | 5,4 % | 11,6 % |

Les résultats présentés dans le tableau V suggèrent que la proportion d'hybrides du groupe 2 (en gris clair) serait de 63,4 %. Dans le même temps, la proportion de souches du groupe 2 représente 64,5 % de la population. Il semble donc que dans la population testée, environ 1 % des hybrides soit des souches de départ n'ayant pas été tuées lors de l'enrichissement à l'éther.

Synthèse à propos de la construction de la population

Pour résumer : il a été possible de construire une population de souches à partir d'un seul hybride de départ. Il a été montré que cette nouvelle population, qui a été générée après 4 phases d'hybridation et de sporulation en masse, est le produit d'un brassage génétique important.

**Sélection des individus d'intérêt dans la population obtenue**

Après vérification de la qualité du brassage, les levures obtenues ont été sélectionnées sur leur capacité à fermenter le xylose tout en résistant aux inhibiteurs. Pour ce faire, elles ont été cultivées pendant 48 heures sur milieu $YFI_1$ puis transférées pour une culture de 72 heures en milieu YFX. (NB. Les compositions des milieux sont indiquées dans le tableau I (*supra*) ou dans le texte qui suit le tableau). Des échantillons de cette seconde population ont été étalés sur un milieu YEG (*supra*). Cette dernière étape a mené, entre autres, à l'isolement de la souche déposée à la CNCM (Collection Nationale de Cultures de Microorganismes, Institut Pasteur, 25 rue du Docteur Roux, 75724 Paris Cedex 15) sous le numéro I-4749.

**Validation de la propagation optimisée**

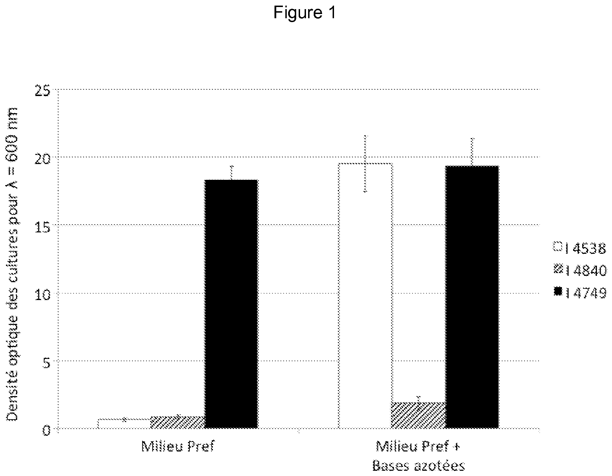

Dans un premier temps, la propagation des souches a été testée sur le milieu Pref, et sur le milieu Pref + bases azotées. Ceci est illustré par la Figure 1. Ces résultats indiquent que :

- la souche I-4538, souche recombinante utilisée pour la présente invention, se propage efficacement sur milieu Pref + bases azotées, mais se propage très mal sur milieu Pref ;

- la souche EGAc1 (I-4839), souche sauvage croisée selon la présente invention avec la souche I-4538, ne se propage efficacement ni sur milieu Pref, ni sur milieu Pref + bases azotées ;
- la souche I-4749 selon l'invention se propage efficacement sur milieu Pref.

**Validation de la propagation sur milieu de type industriel**

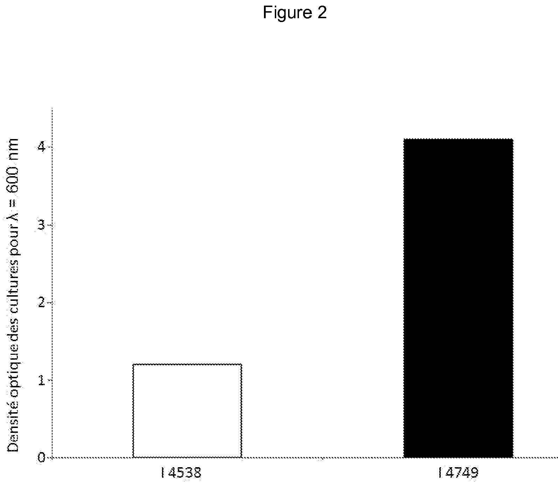

Un milieu YFC (indiqué dans le tableau I *supra*) a été défini comme mimant les conditions d'un milieu industriel de type mélange d'hexoses (ex : glucose, galactose, ...) et de pentoses (ex : xylose, arabinose, ...). La propagation respective des souches I-4538 (souche parente) et I-4749 (souche selon l'invention) a été validée sur ce milieu. La Figure 2 illustre ces résultats et montre une efficacité de propagation plus de deux fois supérieure pour la souche selon l'invention.

**Validation de l'aptitude à la fermentation**

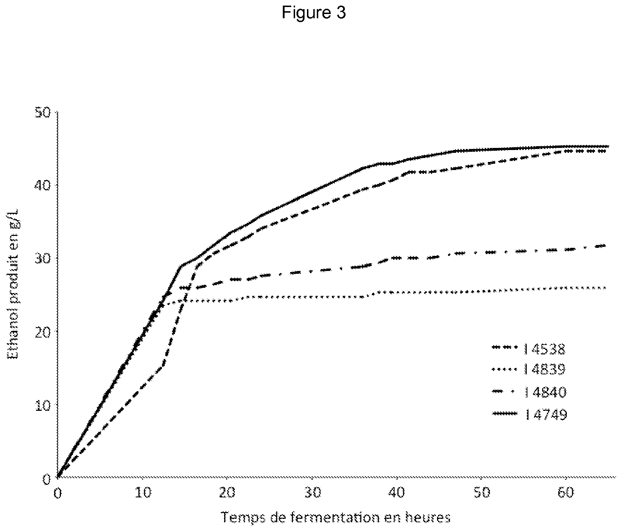

La souche selon l'invention a été utilisée pour effectuer une fermentation dans un milieu proche du milieu réel. Le milieu YFI2 utilisé à cet effet comprend à la fois du glucose et du xylose donc des sucres en C6 et en C5. Le résultat du suivi de perte de masse lors de la fermentation est illustré sur la Figure 3. Dans les cas de toutes les souches mises en oeuvre, la fermentation est biphasique.

Lors de la première phase, les souches EGAc1 (I-4839) et EGAc2 (I-4840) se comportent de la même manière. Il en va de même pour la souche I-4749 obtenue selon la présente invention. En revanche la souche I-4538 est moins rapide lors de cette première phase.

Compte tenu du principe de répression catabolique par le glucose, il est vraisemblable que cette première partie de la fermentation corresponde à la consommation du glucose.

Lors de la seconde phase de la fermentation, il est à noter un important ralentissement pour toutes les souches. Toutefois, les souches les plus performantes sont les souches I-4538 et I-4749. Cette seconde phase correspond probablement à la consommation du xylose ce qui est suggéré par le fait que la souche EGAc1 (I-4839) (de phénotype [Xylose-]) ne fermente pas.

Le bilan est que le meilleur compromis entre toutes les souches mises en oeuvre est bien la I-4749 obtenue selon l'invention.

**Revendications**

1. Procédé d'obtention d'une souche de levure apte à se propager efficacement sur un milieu à faible potentiel nutritif, apte à métaboliser les pentoses et à résister aux inhibiteurs de fermentation présents *ab initio* dans les hydrolysats lignocellulosiques ou formés au cours de la fermentation alcoolique, comprenant les étapes de :

   a) croisement d'une souche de levure recombinante, apte à métaboliser les pentoses et à résister aux inhibiteurs de fermentation, avec une souche de levure sauvage dépourvue de carences, apte à croitre dans un milieu minimum, la souche de levure recombinante comprenant au moins une copie d'un gène exogène de xylose isomérase et au moins une copie surnuméraire d'un gène de D-xylulokinase intégrées au génome dans une région génétiquement liée à un locus d'expression du type sexuel et ségrégeant conjointement,
   b) au moins deux cycles de brassage génétique par sporulation et/ou hybridation aléatoires, sans sélection entre deux,
   c) sélection de la population obtenue à l'étape b) selon un critère d'aptitude des souches à métaboliser le xylose,

d) sélection de la population obtenue à l'étape c) selon un critère d'aptitude des souches à croitre dans un milieu à faible valeur nutritive étant un milieu pauvre en bases azotées comprenant du xylose comme seule source de carbone, une source d'azote minéral, une source de potassium, une source de phosphore, une source de soufre, une source de magnésium, une source de calcium, une source de fer, une source d'oligo-éléments et de l'eau,

sachant que les deux étapes de sélection peuvent être inversées.

**2.** Procédé selon la revendication 1 dans lequel la souche de levure recombinante mise en oeuvre à l'étape a) est la souche de levure déposée à la CNCM (Collection Nationale de Cultures de Microorganismes, Institut Pasteur, 25 rue du Docteur Roux, 75724 Paris Cedex 15) sous le numéro I-4538.

**3.** Procédé selon la revendication 1 ou 2 dans lequel l'étape c) comprend une étape de sélection sur un milieu YFX tel que défini dans le tableau I.

**4.** Procédé selon l'une des revendications 1 à 3, dans lequel l'étape c) comprend les étapes de : mesure du pourcentage de xylose converti en éthanol par au moins un hybride, en condition anaérobie en 60 heures dans un milieu de fermentation comprenant 55 g de glucose et 45 g de xylose par kg dudit milieu, sélection d'au moins un hybride qui convertit au moins 70% du xylose en éthanol en 60 heures.

**5.** Procédé selon l'une des revendications 1 à 4, dans lequel l'étape d) consiste à mesurer la propagation de la souche sur un milieu à faible valeur nutritive comprenant un hydrolysat de protéines et dépourvu de bases azotées.

**6.** Procédé selon la revendication 5 dans lequel le milieu à faible valeur nutritive est le milieu Pref tel que défini dans le Tableau I.

**7.** Procédé selon la revendication 1 comprenant une étape supplémentaire de transformation génétique par addition d'au moins une copie du gène *GAL2.*

**8.** Souche de levure apte à métaboliser les pentoses en résistant aux inhibiteurs de fermentation comprenant au moins une copie d'un gène exogène de xylose isomérase et au moins une copie surnuméraire d'un gène de D-xylulokinase intégrées au génome et liées à un seul des caractères sexuels de la souche, apte à croitre sur un milieu à faible valeur nutritive comprenant un hydrolysat de protéines et dépourvu de bases azotées, susceptible d'être obtenue par le procédé selon l'une des revendications 1 à 6, ladite souche étant de l'espèce *Saccharomyces cerevisiae* et étant déposée à la CNCM (Collection Nationale de Cultures de Microorganismes, Institut Pasteur, 25 rue du Docteur Roux, 75724 Paris Cedex 15) sous le numéro I-4749.

**9.** Souche de levure obtenue à partir de la souche selon la revendication 8, par introduction d'au moins une copie surnuméraire du gène *GAL2.*

**10.** Souche de levure selon la revendication 9, ladite souche étant de l'espèce *Saccharomyces cerevisiae,* et étant déposée à la CNCM (Collection Nationale de Cultures de Microorganismes, Institut Pasteur, 25 rue du Docteur Roux, 75724 Paris Cedex 15) sous le numéro I-4829.

**11.** Culture comprenant la multiplication d'une souche de levure selon l'une des revendications 8 à 10.

**12.** Procédé de production d'au moins un produit de fermentation comprenant les étapes de :

a) fermentation, en condition anaérobie ou semi-anaérobie, d'un milieu comprenant une source de xylose, par une souche de levure selon l'une des revendications 8 à 10 ou d'une culture selon la revendication 11,
b) obtenir de l'éthanol.

**13.** Utilisation d'une souche de levure selon l'une des revendications 8 à 10 ou d'une culture selon la revendication 11 pour la conversion et la métabolisation d'un matériel d'origine végétale comprenant du xylose.

**14.** Utilisation selon la revendication 13 pour produire de l'éthanol.

**Patentansprüche**

1. Verfahren zur Gewinnung eines Hefestammes, der sich in einem Medium mit niedrigem Nährwertpotential wirksam vermehren kann, der in der Lage ist, Pentosen zu metabolisieren und Fermentationsinhibitoren zu widerstehen, die ab initio in Lignocellulose-Hydrolysaten vorhanden sind oder während der alkoholischen Gärung gebildet werden, umfassend die folgenden Schritte:

   a) Kreuzung eines rekombinanten Hefestammes, der in der Lage ist, Pentosen zu metabolisieren und Fermentationsinhibitoren zu widerstehen, mit einem Stamm von Wildhefe, der keine Beeinträchtigungen aufweist und in einem Minimal Medium wachsen kann, wobei der rekombinante Hefestamm mindestens eine Kopie eines exogenen Xylose-Isomerase-Gens und mindestens eine überzählige Kopie eines von D-Xylulokinase-Gens umfasst, die sich im Genom in einer Region befindet, die genetisch verknüpft ist mit einem Expressionslokus sexueller Art und sich gemeinsam aufspaltet,
   b) mindestens zwei Zyklen genetischer Vermischung durch zufällige Sporulation und/ oder Hybridisierung, ohne zwischen beiden zu auszuwählen;
   c) Auswahl der in Schritt b) erhaltenen Population nach einem Eignungskriterium der Stämme zur Xylose-Metabolisierung,
   d) Auswahl der in Schritt c) erhaltenen Population gemäß dem Kriterium inwieweit die Stämme geeignet sind, in einem Medium mit niedrigem Nährwertpotential zu wachsen, bei dem es sich um ein an Stickstoffbasen armes Medium handelt, das Xylose als einzige Kohlenstoffquelle, eine mineralische Stickstoffquelle, eine Kaliumquelle, eine Phosphorquelle, eine Schwefelquelle, eine Magnesiumquelle, eine Kalziumquelle, eine Eisenquelle, eine Quelle für Oligoelemente und Wasser enthält,

   im Wissen, dass die beiden Auswahlschritte auch umgekehrt werden können.

2. Verfahren nach Anspruch 1, wobei der Quelle rekombinante Hefestamm, der in Schritt a) eingesetzt wird, der Hefestamm ist, der hinterlegt ist bei der CNCM (Collection Nationale de Cultures de Microorganismes, Institut Pasteur, 25 rue du Docteur Roux, 75724 Paris Cedex 15) unter der Nummer I-4538.

3. Verfahren nach Anspruch 1 oder 2, wobei der Schritt c) einen Auswahlschritt in einem YFX-Medium ist, wie definiert in Tabelle I.

4. Verfahren nach irgendeinem der Ansprüche 1 bis 3, wobei Schritt c) die folgenden Schritte umfasst: Messung des Prozentsatzes an in Ethanol umgewandelter Xylose durch mindestens ein Hybrid, unter anaeroben Bedingungen, 60 Stunden in einem Fermentationsmedium, umfassend 55 g Glucose und 45 g Xylose pro kg dieses Mediums, Auswahl mindestens eines Hybrids, das mindestens 70 % der Xylose in 60 Stunden in Ethanol umwandelt.

5. Verfahren nach irgendeinem der Ansprüche 1 bis 4, wobei Schritt d) darin besteht, die Vermehrung des Stammes in einem Medium mit niedrigem Nährwertpotential, mit einem Protein-Hydrolysat, das keine Stickstoffbasen enthält zu messen.

6. Verfahren nach Anspruch 5, wobei das Medium mit niedrigem Nährwertpotential, ein Pref-Medium ist, wie definiert in Tabelle I.

7. Verfahren nach Anspruch 1, mit einem zusätzlichen Schritt der genetischen Umwandlung durch Zugabe mindestens einer Kopie des Gens *GAL2*.

8. Hefestamm, der in der Lage ist, Pentosen zu metabolisieren und Fermentationsinhibitoren zu widerstehen, der mindestens eine Kopie eines exogenen Xylose-Isomerase-Gens und mindestens eine überzählige Kopie eines D-Xylulokinase-Gens umfasst, die sich im Genom befindet und genetisch verknüpft sind mit einer einzigen der Eigenschaften sexueller Art des Stammes, der in der Lage ist, sich in einem Medium mit niedrigem Nährwertpotential, mit einem Protein-Hydrolysat, und ohne Stickstoffbasen zu vermehren und durch das Verfahren nach einem der Ansprüche 1 bis 6 erhalten werden kann, bei diesem Stamm handelt es sich konkret um die Spezies *Saccharomyces cerevisiae* und er ist hinterlegt bei der CNCM (Collection Nationale de Cultures de Microorganismes, Institut Pasteur, 25 rue du Docteur Roux, 75724 Paris Cedex 15) unter der Nummer I- 4749.

9. Hefestamm, erhalten aus einem Stamm gemäß Anspruch 8, durch Einführung mindestens einer überzähligen Kopie des Gens *GAL2*.

10. Hefestamm gemäß Anspruch 9, bei diesem Stamm handelt es sich um die Spezies *Saccharomyces cerevisiae,* und er ist hinterlegt bei der CNCM (Collection Nationale de Cultures de Microorganismes, Institut Pasteur, 25 rue du Docteur Roux, 75724 Paris Cedex 15) unter der Nummer I-4829.

11. Kultur, umfassend die Vermehrung eines Hefestamms nach einem der Ansprüche 8 bis 10.

12. Verfahren zur Herstellung mindestens eines Fermentationsproduktes, umfassend die Schritte:

> a) Fermentation, unter anaeroben oder semi-anaeroben Bedingungen, eines Mediums mit einem Xylose-Stamm, durch einen Hefestamm nach einem der Ansprüche 8 bis 10 oder eine Kultur nach Anspruch 11,
> b) Erzeugung von Ethanol.

13. Einsatz eines Hefestamms gemäß einem der Ansprüche 8 bis 10 oder einer Kultur nach Anspruch 11, zur Umwandlung und Metabolisierung eines Xylose enthaltenden Materials pflanzlichen Ursprungs.

14. Einsatz nach Ansprüche 13 zur Erzeugung von Ethanol.


**Claims**

1. A process for obtaining a yeast strain suited to propagating effectively on a medium with low nutritional potential, suited to metabolize pentoses, and with resistance to fermentation inhibitors present *ab initio* in lignocellulosic hydrolysates or formed during alcoholic fermentation, comprising the steps of:

> a) crossing a recombinant yeast strain, suited to metabolize pentoses and to resist to fermentation inhibitors, with a wild yeast strain devoid of deficiencies, suited to grow on a minimum medium, wherein the recombinant yeast strain includes at least one copy of an exogenous xylose isomerase gene and at least one additional copy of a D-xylulokinase gene incorporated into the genome and linked to one of the mating traits of the strain and jointly segregating,
> b) at least two cycles of genome shuffling by random sporulation and/or hybridization, without selection between shuffling,
> c) selecting the population obtained in step b) according to a criterion of ability of the strains to metabolize xylose,
> d) selecting the population obtained in step c) according to a criterion of ability of the strains to grow on a medium of low nutritional value which is poor in nitrogenous bases and which comprises xylose as a sole source of carbon, a source of mineral nitrogen, a source of potassium, a source of phosphorous, a source of sulphur, a source of magnesium, a source of calcium, a source of iron, a source of trace elements and water,

> noting that the two steps of selection can be reversed.

2. The process according to claim 1, wherein the recombinant yeast strain implemented in step a) is the yeast strain deposited at the CNCM (National Collection of Microorganism Cultures, Institut Pasteur, 25 rue du Docteur Roux, 75724 Paris Cedex 15) under number 1-4538.

3. The process according to claim 1 or 2, wherein step c) includes a step of selection on a YFX medium as defined in Table I.

4. The process according to any of claims 1 to 3, wherein step c) comprises the steps of: measuring the percentage of xylose converted into ethanol by at least one hybrid, under anaerobic conditions over 60 hours in a fermentation medium comprising 55 g of glucose and 45 g of xylose per kg of said medium and selecting at least one hybrid that converts at least 70% of the xylose into ethanol in 60 hours.

5. The process according to any of claims 1 to 4, wherein step d) consists in measuring the propagation of the strain on a medium of low nutritional value that comprises a protein hydrolysate and is devoid of nitrogenous bases.

6. The process according to claim 5, wherein the medium of low nutritional value is the Pref medium as defined in Table I.

7. The process according to claim 1, further comprising a step of genetic transformation by the addition of at least one copy of the *GAL2* gene.

8. Yeast strain suited to metabolize pentoses and with resistance to fermentation inhibitors which includes at least one copy of an exogenous xylose isomerase gene and at least one additional copy of a D-xylulokinase gene incorporated into the genome and linked to one of the mating traits of the strain, suited to grow on a medium of low nutritional value that comprises a protein hydrolysate and is devoid of nitrogenous bases, obtainable by the process according to any of claims 1 to 6, said yeast being of the species *Saccharomyces cerevisiae,* and being deposited at the CNCM (National Collection of Cultures of Microorganisms, Pasteur Institute, 25 rue du Docteur Roux, 75724 Paris Cedex 15) under number I-4749.

9. Yeast strain obtained from the strain according to claim 8, by introducing at least one additional copy of the *GAL2* gene.

10. Yeast strain according to claim 9, said strain being of the species *Saccharomyces cerevisiae* and being deposited at the CNCM (National Collection of Cultures of Microorganisms, Pasteur Institute, 25 rue du Docteur Roux, 75724 Paris Cedex 15) under number 1-4829.

11. Culture comprising the multiplication of a yeast strain according to any of claims 8 to 10.

12. A method for producing at least one fermentation product comprising the steps of:

    a) fermenting, in anaerobic or semi-anaerobic conditions, a medium comprising one source of xylose, by a yeast strain according to any of claims 8 to 10 or a culture according to claim 11,
    b) obtaining ethanol.

13. Use of a yeast strain according to any of claims 8 to 10 or of a culture according to claim 11 for the conversion and the metabolism of a material of plant origin comprising xylose.

14. Use according to claim 13 for producing ethanol.

Figure 1

Figure 2

Figure 3

## EP 3 108 016 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010000464 A1
- WO 2012072793 A1
- WO 2013178915 A1
- WO 2011079388 A
- WO 2013178918 A1

**Littérature non-brevet citée dans la description**

- **GRANGE et al.** *Appl. Microbiol. Biotechnol.,* 2010, vol. 87, 1195-1208
- **JORDAN et al.** *Biochem. J.,* 2012, vol. 442, 241-252
- **DEMEKE et al.** *Biotechnology for Biofuels,* 2013, vol. 6, 89
- **BELLISSIMI E ; RICHARDS C.** Yeast propagation. In The alcohol textbook, a reference for the beverage, fuel and industrial alcohol industries. University Press, 2009, 145-159
- Sporulation and Hydridization of Yeast. **R.R. FOWELL.** The Yeasts. Academic Press, 1969, vol. 1
- **HOU.** *Biotechnol. Lett.,* 2009, vol. 31, 671-677
- **HAMACHER et al.** *Microbiology,* 2002, vol. 148, 2783-2788
- **G. REED ; T.W. NAGODAWITHANA.** Yeast Technology. Van Nostrand Reinhold, 1991
- **WILLIAMS.** *J. Bacteriol.,* 1936, vol. 32 (6), 589-597
- **HO ; MILLER.** *Can. J. Microbiol.,* 1978, vol. 24 (3), 312-320
- **DAWES ; HARDIE.** *Mol. Gen. Genet.,* 1974, vol. 131 (4), 281-289

26